Europäisches Patentamt

European Patent Office

Office européen des brevets

(11) Veröffentlichungsnummer: **0 082 510**
**B1**

(12) **EUROPÄISCHE PATENTSCHRIFT**

(45) Veröffentlichungstag der Patentschrift: 02.03.88

(51) Int. Cl.⁴: **A 61 M 1/00**

(21) Anmeldenummer: **82111761.1**

(22) Anmeldetag: **17.12.82**

(54) **Saugflasche für medizinische Zwecke.**

(30) Priorität: **21.12.81 DE 3150500**

(43) Veröffentlichungstag der Anmeldung:
**29.06.83 Patentblatt 83/26**

(45) Bekanntmachung des Hinweises auf die
Patenterteilung:
**02.03.88 Patentblatt 88/09**

(84) Benannte Vertragsstaaten:
**CH DE FR GB IT LI SE**

(56) Entgegenhaltungen:
**DE-A-1 810 801**
**DE-A-2 035 241**
**US-A-3 646 935**
**US-A-3 719 197**
**US-A-3 768 478**
**US-A-3 809 085**
**US-A-3 830 238**
**US-A-3 845 765**
**US-A-4 135 515**

(73) Patentinhaber: **Lauterjung, Friedrich Gerd**
**Schallstrasse 6**
**D-5000 Köln 41 (DE)**

(72) Erfinder: **Lauterjung, Friedrich Gerd**
**Schallstrasse 6**
**D-5000 Köln 41 (DE)**

(74) Vertreter: **Rieder, Hans-Joachim, Dr.**
**Corneliusstrasse 45 Postfach 11 04 51**
**D-5600 Wuppertal 11 (DE)**

EP 0 082 510 B1

Courier Press, Leamington Spa, England.

**Beschreibung**

Die Erfindung betrifft eine Saugflasche gemäß dem ersten Teil des Anspruchs 1.

Bei den bekannten Lösungen dieser Art (US—A—3 845 765) ist der Wegwerfbeutel eingeklemmt zwischen Deckel und Gefäß der Saugflasche. Dies ist dichtungstechnisch problematisch und verlangt vor allem hohe Spannkräfte zwischen Deckel und Saugflaschengefäß, um selbst bei unvermeidbarer Faltenbildung im Beutelmaterial eine genügende Dichtigkeit zu gewährleisten. Darüber hinaus ist bei diesen Lösungen auch von Nachteil, daß neben dem Drainageschlauch in den Wegwerfbeutel auch noch eine Luftabsaugleitung ragt, die zur Innenkammer der Saugflasche führt, von welcher dann an der gegenüberliegenden Saugflaschenseitenwand der Anschlußstutzen für den zur Pumpe reichenden Schlauch wegführt. Das ist nicht nur aufwendig, sondern erfordert auch aus Sicherheitsgründen, daß stets die Saugflasche selbst einschließlich Deckel und ein erheblicher Teil des Schlauchsystemes sterilisiert werden muß, bevor ein neurer Einsatz stattfindet. Um das Einklemmen des Wegwerfbeutelrandes zwischen Deckel und Unterteil der Saugflasche zu vermeiden, hat man innerhalb der gleichen Literaturstelle auch schon vorgeschlagen, den Wegwerfbeutel an ein Gestell zu hängen, welches in die Innenkammer der Saugflasche gestellt wird. Diese verlangt aber dichtende Anschlüsse, dit mit dem Beutelmaterial verbunden werden müssen, was nicht nur den Preis der Wegwerfbeutel erheblich erhöht, sondern auch Herstellungs- und handhabungstechnisch erhebliche Nachteile bringt. Solche Nachteile werden noch dadurch vergrößert, daß die dabei vorgesehene Beutelhalterung auf eine geraden Linie erfolgt und nicht auf einer Kreislinie wie bei der Einspannung der Wegwerfbeutels über den Flaschenrand.

Bei anderen bekannten Lösungen (DE—A— 1 810 801) ist der Wegwerfbeutel ebenfalls mit seiner oberen Kante linienförmig an der Unterseite eines Deckels der Saugflasche aufgehängt. Der Drainageschlauch ist auch dort mit dem Beutelrand fest und dicht verbunden und durch ein zentrisches Loch des Deckels hindurchgeführt. In möglichst großem Abstand von diesem Loch sitzt ein Ventil-Anschlußstutzen zum Evakuieren der Saugflächen-Innenkammer, was erlaubt, die Innenkammer zu evakuieren und so die Saugflasche auch ohne direkte Pumpenverbindung zu betreiben. Die geradlinige Aufhängung des Beutels ist vorteilhaft für das Ausgangsstadium in der Arbeitsweise, weil der Beutel in dieser Form weitgehendst luftleer flachgedrückt Wand an Wand liegen muß. Die geradlinige Aufhängung ist jedoch nachteilig insofern, als die Aufhängestellen eine hohe spezifische Hang- und Spreizlast aufnehmen müssen und das Verhältnis zwischen Größe der Beutelwandung in Relation zum max. erreichbaren Beutelinnenvolumen weit von dem optimalen Wert entfernt liegt; ferner kann der selbst vollständig gefüllte Beutel das Volumen der Innenkammer der Saugflasche nicht annähernd ausfüllen, so daß diese freibleibenden Volumina überflüssigerweise evakuierte Teil-Räume darstellen, was wiederum einen erheblichen Pumpen-Energieverlust bedeutet. Außerdem geht dabei unnötig Pump-Zeit verloren.

Aufgabe der vorliegenden Erfindung ist es, eine gattungsgemäße Saugflasche so auszubilden, daß mit den gleichen Mitteln einerseits eine größere Bedienungssicherheit gegeben ist und andererseits eine vereinfachte Handhabung bei ausreichend sterilen Bedingungen unter geringstmöglichem Aufwand.

Gelöst ist diese Aufgabe durch die im zweiten Teil des Anspruchs 1 angegebenen Merkmale. Die abhängigen Ansprüche 2 bis 5 stellen vorteilhafte Weiterbildungen dar.

Zufolge dieser Ausgestaltung ist eine Saugflasche für medizinische Zwecke geschaffen, welche bei vergrößerter Bedienungssicherheit einfacher zu handhaben und preislich günstiger herzustellen ist. Die Halterung des Wegwerfbeutels ist optimal auch hinsichtlich der Evakuierung: Überflüssigerweise zu evakuierende Freiräume sind auf ein Minimum reduziert, weil die ringförmige Einspannstelle des Beutelrandes eine wesentlich weitgehendere Ausfüllung der Innenkammer der Saugflasche erlaubt als eine geradlinienförmige Aufhängung des Beutels. Dabei wurde gefunden, daß der Volumenverlustfaktor, welcher aus der Faltung des Wegwerfbeutels im Übergangsbereich zwischen ringförmiger Einspannung und notwendiger Ausgangsflachform ohne weiteres in Kauf genommen werden kann unter Berücksichtigung des erreichten Vorteiles der besseren Ausfüllung der Innenkammer durch den gefüllten Wegwerfbeutel und auch unter Berücksichtigung der Tatsache, daß diese, aus solchen Faltungen resultierenden Freiräume dadurch relativ klein ausfallen, weil man wegen der Aufhängung des Beutels an seinem ganzen oberen Rand in Verbindung mit der dadurch eingeleiteten gleichmäßigen Hang- und Spreiz-Last mit relativ wesentlich dünnerem Beutelmaterial auskommt. Die Zuordnung Beutel/Deckel/Drainageschlauch braucht dabei nicht mehr fabrikmäßig zu erfolgen, sondern kann evtl. sogar vom Krankenhauspersonal durchgeführt werden, was eine vereinfachte Zulieferung und Lagerhaltung bedeutet. Der Schlauch braucht nicht dichtend zum Beutelmaterial und mit diesem verklebt bzw. verschweißbar zu sein; der Deckel der Flasche ist das Verbindungselement zwischen Schlauch und Beutel. Der Deckel kann -vor allem wenn er nicht mehr das teure Ventil enthältzugleich zum Wegwerfteil werden. In den Wegwerfbeutel gelangt nur des Drainageschlauch und kein Luftabsaugschlauch. Das Vorratsvakuum der Innenkammer bringt die Saugenergie zum Füllen des Wegwerfbeutels. Der Ventil-Anschlußstutzen sitzt am Boden. Diese bedeutet, daß bei Benutzung des in der Größe passenden Wegwerfbeutels nicht die Möglichkeit besteht, daß sich die Beutelwand beim Evakuieren vor den Ventil-Anschlußstutzen legt und den Evakuierungsvorgang frühzeitig stoppt. Deshalb kann die Evakuierung auch mit sehr hohen Saugleistungen vorgenommen werden, was eine Zeitersparnis bedeutet.

Bei noch anderen Saugflaschen ist es bereits bekannt (US—A—3 719 197), den oberen Rand des Beutels längs einer ringförmigen Linie aufzuhängen. Diese Vorrichtungen arbeiten jedoch nicht mit einem Vorrats-Vakuum. Zusätzlich zum Drainageschlauch mündet auch dort vielmehr in den Wegwerfbeutel gleichzeitig ein Pumpenschlauch, durch der ein Unterdruck erzeugt wird. Während der gesamten Arbeitsweise ist diese Saugverbindung zur Pumpe aufrechtzuerhalten. Um zu vermeiden, daß sich der Wegwerfbeutel durch den Unterdruck flach saugt, besitzt die Saugleitung der Pumpe eine Abzweigungsstelle, die an der Mantelwand der Saugflasche, also außenseitig des Wegwerfbeutels in die Innenkammer derselben einmündet. Dadurch ist ein Unterdruck-Gleichgewicht gehalten zwischen dem Innerem des Wegwerfbeutels und seinem Umgebungsraum. Wiederum ist der Wegwerfbeutel wegen der notwendigen direkten Schlauchanschlüsse sehr teuer und es besteht auch hier die Gefahr, daß Sekrete in den Innenraum der Flasche gelangen.

Ein weiterer Vorteil der Erfindung ist darin zu sehen, daß der Anschlußstutzen sich in einen bis zum Beutelboden reichenden Schlauch fortsetzt. Dieser steift den Beutel zuordnungserleichternd aus. Eine günstige Bauform erhält die Saugflasche dadurch, daß der Evakuier-Ventilanschlußstutzen von einem Standfuß-Kragen überragt ist. Der Standfuß-Kragen bringt für die Anschlußstelle eine schützende Kammer. Die bodenseitige Lage der Anschlußstelle ermöglicht es, die Saugflaschen in aufstehender Anordnung, bspw. unter Zwischenschaltung einer Steckkupplung, praktisch batterieweise zu evakuieren. Eine schnelle, bequeme Deckelzuordnung läßt sich mit einfachen Mitteln dadurch bewirken, daß der Saugflaschen-Deckel in einer Steck-Dreh-Verbindung auf der Saugflasche befestigt ist durch Untergriff randseitiger Zonen unter Vorsprünge des Saugflaschen-Randes. Dabei ist es günstig, wenn den randseitigen Zonen ansteigende Klemmflächen zugeordnet sind, die von Einstecknischen des Saugflaschen-Deckels ausgehen. Endlich besteht eine auch in Extremfällen noch störungsfrei arbeitende Saugflasche darin, daß im Abstandsbereich Löcher für eine Vakuumanzeige und einen Lufteinlaß vorgesehen sind.

Nachstehend wird ein Ausführungsbeispiel der Erfindung anhand der Figuren 1—4 erläutert. Es zeigt

Fig. 1 einen Längsschnitt durch eine einen Wegwerfbeutel aufnehmende Saugflasche mit zugehörigem Drainageschlauch (bei zur besseren Verdeutlichung der Funktionselemente versetzter Deckelschnittebene),

Fig. 2 die Draufsicht hierzu,

Fig. 3 den Schnitt gemäß Linie III—III Fig. 1 und

Fig. 4 die Seitenansicht des Saugflaschen-Dekkels unter Verdeutlichung einer der ansteigenden Klemmflächen.

Die im wesentlichen zylindrisch geformte Saugflasche 1 ist an ihrem oberen Ende mittels eines Deckels 16 dicht verschließbar.

Ein Standfuß-Kragen 6 überragt die Bodenfläche 7 der Saugflasche. Beide, die Saugflasche 1 und der Standfuß-Kragen 6 bestehen aus transparentem Material. In zentraler Anordnung ist an der Bodenfläche 7 ein Ventilanschlußstutzen 8 zum Evakuieren der Luft vorgesehen. Durch den Standfuß-Kragen 6 liegt der Ventil-Anschlußstutzen 8 in geschützter Anordnung.

Der den oberen Abschluß der Saugflaschen-Innenkammer K bildende Deckel 16 tritt mit seiner Randzone gegen einen flexiblen Dichtungsring 5 am Öffnungsrand der Saugflasche 1.

In etwa zentraler Anordnung besitzt der Deckel 16 eine Drainageschlaucheintrittsstelle. Letztere nimmt einen Schlauch 22 dichtend auf. Dieser ragt in einen in der Innenkammer K angeordneten, vom Unterdruck volumenvergrößerbaren Wegwerfbeutel 21. Sein äußeres, über einen Stutzen 16″ des Deckels 16 vorstehendes Ende bildet einen Drainageschlauch-Anschlußstutzen 20. Der Schlauch 22 reicht nahezu bis zum Beutelboden. An das untere Schlauchende 22′ schließt sich ein Ventil 23 an. Dessen endseitige Dichtungslippen verhindern einen Rückfluß der in den Wegwerfbeutel 21 gelangenden Flüssigkeit.

Der Drainageschlauch 27 wird über ein Verbindungsstück 28 mit einem in der Körperhöhlung unterzubringenden Drainageschlauch-Abschnitt 29 verbunden. Das andere Ende des Drainageschlauches 27 trägt einen von einer Schlauchklemme 30 verschließbaren Schlauchabschnitt 31, von welchem ein Verbindungs-Schlauchstück 32 ausgeht. Letzteres wird mit dem Anschlußstutzen 20 des Schlauches 22 gekuppelt. Danach ist eine weitere Schlauchklemme 25 zwischen Schlauch 22 und Schlauchstück 32 in ihre Freigabestellung zu bringen. Das Verschlußstück 25 sitzt auf einem auf das Schlauchende 20 aufgesteckten Schlauchabschnitt 20‴. Nachdem auch die Schlauchklemme 30 in die Freigabestellung gebracht wurde, ist der Weg vom Drainageschlauch-Abschnitt 29 zum Wegwerfbeutel 21 frei. Durch den in der Innenkammer K vorhandenen Unterdruck tritt nun eine Volumenvergrößerung des Wegwerfbeutels 21 ein bei gleichzeitig auftretender Saugwirkung.

Bezüglich der bodenseitigen Ventil-Anschlußstelle zum Evakuieren der Innenkammer K handelt es sich um ein Kugelventil, welches, unterstützt durch eine Feder 8′, bei in der Innenkammer herrschendem Unterdruck gegen seine Ventilsitzfläche 8″ tritt. Unter Saugwirkung hebt sich das Ventil unter Überwindung der Federkraft von seiner Sitzfläche ab. Der Ventil-Anschlußstutzen 8 liegt im Abstand zum Wegwerfbeutel 21. Er steht beiderseits vor und weist einen sich kammerseitig schwach verjüngenden Abschnitt auf, gegen den klemmsitzartig ein entsprechend kegelstumpfartiger Abschnitt des Ventilgehäuses tritt. Das Vakuum begünstigt den festen Klemmsitz der konischen Flächen noch.

Die evakuierte Saugfläche kann leicht von der stationären Unterdruckquelle abgekuppelt werden. Es ergibt sich ein bequemer, ortsveränderlicher Einsatz ohne dauernd mit der Pumpe verbunden zu sein. Die Konditionierung ist nach Entfernen des Wegwerfbeutels und Einsetzen eines neuen bequem möglich. Dabei liegt auch der Vorteil vor, daß, nachdem das Vakuum einer Saugflasche erschöpft ist, man eine entsprechend vorbereitete, auf Vorrat gehaltene neue Flasche ansetzt usw.

Die Zuordnung des Wegwerfbeutels 21 am Deckel 16 der Flasche erfolgt unter Verwendung einer Klemmverbindung. Hierzu dient ein Ring 36. Durch diesen hindurch ist der obere Rand 21' des Wegwerfbeutels 21 von unten her eingezogen und nach außen hin über den etwa quadratischen Ringquerschnitt gefaltet. Hierdurch liegt der obere Rand 21' an der Ringinnenfläche, der Ringoberseite und der Ringaußenfläche als der eigentlichen Klemmhaltefläche an. Der Rand überragt etwas die Unterseite des Ringes 36. Die Ringaußenfläche kann sich in Steckrichtung leicht verjüngen. Der so provisorisch festgelegte Wegwerfbeutel wird nun unter Eindrücken des Ringes 36 in eine passende Vertiefung 37 an der Unterseite 16' des Saugflaschen-Deckels 16 endgültig fixiert. Die Breite der Vertiefung berücksichtigt das für eine hierbei auftretende Klemmbefestigung erforderliche lichte Maß. Es liegt etwas unterhalb der Wandungsdicke des Wegwerfbeutels, wodurch dem Beutelmaterial dort zugleich die Funktion einer Dichtungseinlage zukommt, was den Befestigungshalt optimiert.

Der Ring 36 nimmt eine konzentrische Lage zum im Grunde kreisrunden Deckel 16 ein. Unter Berücksichtigung der Zylinderform der Flasche liegt auch in Bezug hierauf die konzentrische Zuordnung vor. Der Ring 36 ist so bemessen, daß er etwa im halben Abstand zur Saugflaschen-Innenwand 1' verläuft, also in einem Abstand, der dem Ringradius etwa entspricht. Dies ist unter Berücksichtigung der auftretenden Hanglast günstig. Der Halsbereich des Wegwerfbeutels wird weniger beansprucht.

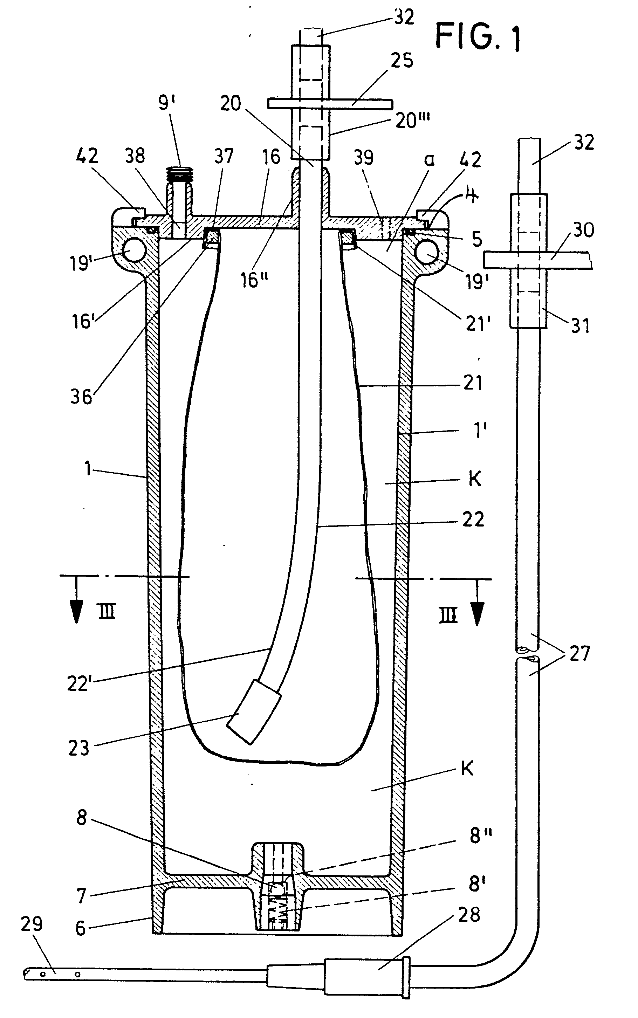

Innerhalb des ringförmig vorliegenden Abstandsbereichs a zwischen Saugflaschen-Innenwand 1' und Außenwand des Wegwerfbeutels 21 sind am Deckel 16 Löcher 38 und 39 vorgesehen. Dem Loch 38 ist der Volumenanzeiger 9' zugeordnet. Das in Fig. 1 versetzt gezeichnete Loch 39 dient dagegen als Lufteinlaß. Das Loch 39 wird von einem außenseitig des Deckels befestigten Ventil-Lappen 40 verschlossen. Es handelt sich um einen Klebestreifen. Nach Abziehen desselben strömt die Außenluft in die Innenkammer K ein, dies bspw. um den Rest-Unterdruck zu beseitigen oder das maximale Vakuum zu reduzieren. Diese ist von Bedeutung für die individuelle Anpassung an unterschiedliche Operationsfälle.

Der Saugflaschen-Deckel 16 wird im Wege einer Steck-Dreh-Verbindung zugeordnet. Er weist dazu an diametral einander gegenüberliegenden Bereichen Einstecknischen 41 auf. Letztere erlauben den freien Steckdurchtritt im Bereich ebenfalls diametral einander gegenüberliegender Vorsprünge 42 des Saugflaschen-Randes, dessen stirnseitiger Öffnungsrand 4 den vorstehenden flexiblen Dichtungsring 5 trägt. Der hier dichtend aufliegende Randbereich ist gegenüber der zentralen, mit dem Innenquerschnitt der zylindrischen Flasche maßlich übereinstimmenden Deckelfläche ausgedreht, so daß diese vorspringende Zone den Deckel 16 gegen Querverlagerung sichert.

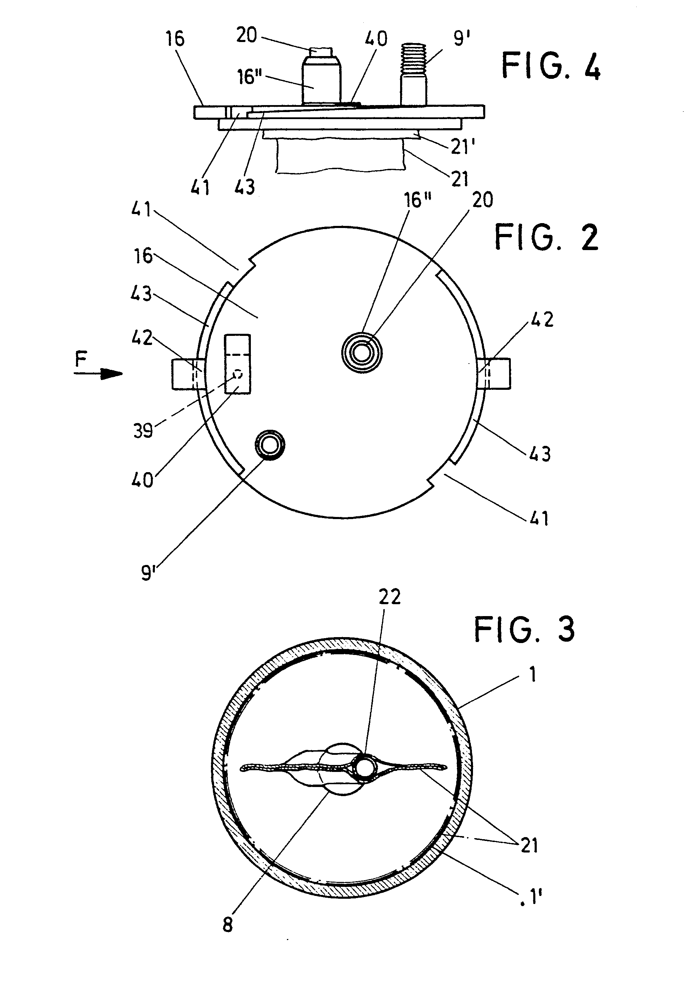

Die in Drehrichtung (Rechtsdrehung) an die Einstecknischen 41 anschließenden randseitigen Zonen untergreifen die den Deckelrand oberseitig hakenförmig überlagernden Vorsprünge 42. Wie Fig. 4 entnehmbar, sind diese randseitigen, sich etwa über einen Viertelkreis erstreckenden Zonen an ihrer Oberseite zu ansteigenden Klemmflächen 43 ausgebildet. Letztere beginnen auf etwa halber Höhe der Einstecknischen und laufen auf der Oberseite des Deckels aus, gegebenenfalls unter Bildung einer End-Anschlagschulter. Die Klemmung setzt nach etwa einem Drehbereich von 25° ein, so daß ein völliges Überlaufen der Klemmflächen praktisch ausgeschlossen ist.

Weitere Sicherungsmaßnahmen für den Deckel sind nicht erforderlich, da das erzeugte Vakuum den Deckel fest gegen die Dichtung 5 zieht. Das Öffnen läßt sich erst nach Druckausgleich bewirken.

**Patentansprüche**

1. Saugflasche für medizinische Zwecke mit evakuierbarer Innenkammer (K) und einem in der Innenkammer (K) hängend angeordneten Wegwerfbeutel (21), in dessen Inneres ein Anschlußstutzen (20) eines Drainageschlauches (27) mündet und dessen Rand (21') am Deckel (16) der Saugflasche (1) vakuumdicht eingespannt ist, dadurch gekennzeichnet, daß der obere Rand (21') des vom Unterdruck der Innenkammer (K) volumenvergrößerbaren Wegwerfbeutels (21) um einen Ring (36) gefaltet ist, welcher in eine Vertiefung (37) an der Unterseite (16') des Saugflaschen-Deckels (16) eingeklemmt ist und welcher im Abstand zur Saugflaschen-Innenwand (1') verläuft.

2. Saugflasche nach Anspruch 1, dadurch gekennzeichnet, daß der Saugflaschen-Deckel (16) in einer Steck-Dreh-Verbindung auf der Saugflasche (1) befestigt ist durch Untergriff randseitiger Zonen unter Vorsprünge (42) des Saugflaschen-Randes.

3. Saugflasche nach Anspruch 1, dadurch gekennzeichnet, daß ein Drainageschlauch-Anschlußstutzen (20) sich in einen bis zum Beutelboden reichenden Schlauch (22) fortsetzt und das untere Schlauchende (22') mit einem Ventil (23) versehen ist.

4. Saugflasche nach Anspruch 1, gekennzeichnet durch eine bodenwandseitige (7) Anordnung eines Ventil-Anschlußstutzens (8), der von einem Standfuß-Kragen (6) überragt ist.

5. Saugflasche nach Anspruch 1, dadurch gekennzeichnet, daß im Abstandsbereich (a) Löcher (38, 39) für eine Vakuumanzeige (9') und einen Lufteinlaß vorgesehen sind.

**Revendications**

1. Flacon d'aspiration pour applications médicales, comportant une chambre intérieure (K) susceptible d'être évacuée et une poche jetable (21) suspendue dans ladite chambre intérieure (K) et dans laquelle débouche un raccord de connexion (20) d'un tuyau de drainage (27), le bord (21') de ladite poche étant assujetti de manière étanche sur le couvercle (16) du flacon d'aspiration, caractérisé en ce que le bord supérieur (21') de la poche jetable (21) dont le volume est susceptible d'être agrandi par l'effet d'une dépression créée dans ladite chambre intérieure (K) est replié autour d'une bague (36) qui est coincée dans un évidement (37) prévu sur la face inférieure (16') du couvercle (16) dudit flacon d'aspiration, et qui s'étend à une certaine distance de la paroi intérieure (1') du flacon d'aspiration.

2. Flacon d'aspiration selon la revendication 1, caractérisé en ce que le couvercle (16) dudit flacon (1) est fixé sur celui-ci par une connexion à enfichage et rotation (à baïonnette) de façon telle que des zones marginales viennent s'accrocher sous des saillies (42) du bord dudit flacon d'aspiration.

3. Flacon d'aspiration selon la revendication 1, caractérisé en ce qu'un raccord de connexion (20) du tuyau de drainage est prolongé par un tuyau (22) plongeant jusqu'au fond de la poche, et en ce que l'extrémité inférieure (22') dudit tuyau comporte une valve (23).

4. Flacon d'aspiration selon la revendication 1, caractérisé en ce qu'il comporte, du côté de la paroi du fond (7), un raccord de connexion (8) de valve, cependant qu'un col formant pied (6) s'étend au-delà du niveau dudit raccord de valve.

5. Flacon d'aspiration selon la revendication 1, caractérisé en ce que des passages (38, 39) pour un indicateur de vide (9') et pour une admission d'air sont prévus dans la zone définie par ladite distance (a).

**Claims**

1. Suction bottle for medical purposes with inner chamber (K) capable of evacuation and a disposable bag (21) arranged hanging in the inner chamber (K), in the inside of which bag a connection stub (20) of a drainage tube (27) opens out and the rim (21') of which bag is clamped vacuum tightly at the lid (16) of the suction bottle (1), characterized in that the upper rim (21') of the disposable bag (21), which can be increased in volume by the negative pressure of the inner chamber (K), is folded around a ring (36), which is clamped into a recess (37) on the underside (16') of the suction bottle lid (16) and which runs at a distance from the suction bottle inner wall (1').

2. Suction bottle according to Claim 1, characterized in that the suction bottle lid (16) is fastened on the suction bottle (1) in a push on and turn connection and by zones on the rim side engaging underneath projections (42) of the suction bottle rim.

3. Suction bottle according to Claim 1, characterized in that a drainage tube connection stub (20) continues into a tube (22) going down to the bottom of the bag and the lower tube end (22') is provided with a valve (23).

4. Suction bottle according to Claim 1, characterized by an arrangement at the bottom wall (7) of a valve connection stub (8), around which a base collar (6) projects.

5. Suction bottle according to Claim 1, characterized in that holes (38, 39) are provided in the spacing area (a) for a vacuum indicator (9') and an air inlet.

0 082 510

FIG. 1

0 082 510

FIG. 4

FIG. 2

F

FIG. 3